# EUROPEAN PATENT APPLICATION

(11) **EP 4 548 929 A1**
(43) Date of publication of application: **07.05.2025**
(21) Application number: 23830251.7
(22) Date of filing: 27.06.2023
(51) Int. Cl.: A61K 39/395, A61K 33/243, C07K 16/30, A61P 35/00

(54) **PHARMACEUTICAL COMPOSITION COMPRISING MIXED ANTIBODY OF ANTI-CTLA4 AND ANTI-PD1 AND THERAPEUTIC USE THEREOF**

(30) Priority: 28.06.2022 CN 202210739031; 06.12.2022 CN 202211569750
(71) Applicant: QILU PHARMACEUTICAL CO., LTD., Jinan, Shandong 250100 (CN)
(72) Inventor: KANG, Xiaoyan, Jinan, Shandong 250100 (CN); FANG, Lianghua, Jinan, Shandong 250100 (CN); WANG, Huayuan, Jinan, Shandong 250100 (CN); FU, Yan, Jinan, Shandong 250100 (CN); LIU, Qianqian, Jinan, Shandong 250100 (CN); HAN, Cuicui, Jinan, Shandong 250100 (CN); XUE, Shilin, Jinan, Shandong 250100 (CN); WU, Ting, Jinan, Shandong 250100 (CN)
(74) Representative: Bandpay & Greuter
(86) International application number: PCT/CN2023/102765
(87) International publication number: WO 2024/002074

(57) **Abstract**

Disclosed in the present invention are a pharmaceutical composition comprising a mixture of antibodies of anti-CTLA4 and anti-PD1, a pharmaceutical composition comprising the mixture of antibodies and a chemotherapeutic drug, and use of the above pharmaceutical composition in treating non-small cell lung cancer.

## Description

### Technical Field

The present disclosure relates to the treatment of cancer, particularly non-small cell lung cancer, including immunotherapy and combination therapy. More specifically, the present disclosure relates to the use of a pharmaceutical composition comprising a mixture of antibodies of anti-CTLA4 and anti-PD1 for treating non-small cell lung cancer at stage II-IIIB after complete surgical resection, or locally advanced or metastatic non-small cell lung cancer that is negative for PD-L1 expression (PDL1 TPS<1%), has no epidermal growth factor receptor (EGFR)-sensitive mutation or altered anaplastic lymphoma kinase (ALK) gene translocation, and has not been subjected to a systemic anti-tumor therapy.

### Technical Background

According to the data from GLOBOCAN in 2020, the incidence rate of lung cancer ranks second among cancers worldwide with 2.2 million cases, and its mortality rate ranks first with 1.8 million lung cancer deaths worldwide. In China, its incidence rate and mortality rate are in the first place, and new lung cancer cases and deaths are 816 thousand and 715 thousand, respectively.

Non-small cell lung cancer (NSCLC) accounts for approximately 85% of all lung cancers, and includes two major pathological types, squamous cell carcinoma and non-squamous cell carcinoma. Non-squamous NSCLC accounts for about 60% of NSCLC, with lung adenocarcinoma being the most common type, accounting for about 40% of NSCLC. In lung adenocarcinoma, approximately 10% of Caucasian patients and 40-60% Asian patients carry EGFR sensitive mutations, mainly including a deletion in EGFR exon 19 and a mutation in L858R in exon 21. There are also mutations in genes such as ALK and ROS1.

About 30% of all NSCLC patients were diagnosed with resectable early NSCLC at initial diagnosis. Surgery is the primary treatment for patients with early NSCLC; and adjuvant chemotherapy is a common treatment for patients with surgically resectable locally advanced NSCLC. In the population receiving a post-operative adjuvant therapy, 20%-30% of patients who died within 5 years were at stage I, 50% at stage II, and 60% at stage IIIA. However, the 5-year survival rate decreases from 90% for stage IA1 of resection to 41% for stage IIIA, indicating micrometastasis in some patients at the time of surgical resection, resulting in treatment failure. At present, for immunotherapy of this part of the population, only Atezolizumab has been approved in China, with a low patient access. There is an urgent need for more kinds of effective medicines.

For patients without any genetic variations (including EGFR mutation or ALK gene rearrangement, also referred to as altered ALK gene translocation) in tumor, immune checkpoint inhibitors (ICIs) in combination with platinum-containing chemotherapy have become a first-line standard treatment. By taking studies including the Keynote-042 study together, it is shown from the data that the positive rate of PD-L1 expression is about 50~60%, where only about 1/3 shows high expression, while patients with negative PD-L1 expression (defined as a tumor proportion score [TPS]<1%) account for about 40~50% of NSCLC patients in China and are a broad treatment population.

Although immune checkpoint inhibitors in combination with platinum-containing chemotherapy have become the first-line standard treatment for advanced, driver-gene-negative NSCLC, there are significant differences in efficacy among populations with different PD-L1 expression levels, and in particular, there is lower efficacy in patients with negative expression of PD-L1. Under the same treatment regimen, the median PFS (mPFS) in patients with negative expression of PD-L1 was 3 months or more lower than that of patients with high PD-L1 expression (TPS≥50%), and the median OS (mOS) was 6 months or more lower than that of patients with high PD-L1 expression (TPS≥50%). For example, in the Keynote-189 study, patients with negative and high PD-L1 expression have mPFSs of 6.1 months and 9.4 months, respectively, and mOSs of 17.2 months and 27.7 months, respectively. NSCLC patients with negative PD-L1 expression and negative driver genes have a far unmet therapeutic need.

Established results suggest that dual immunotherapeutic drugs (combination of two immune checkpoint inhibitors) may have a survival benefit in tumor patients. However, dual immunotherapeutic drugs in combination with chemotherapy have not been approved in China at present. Immunotherapeutic drugs approved in China are used for locally advanced or metastatic NSCLC in the whole population or those with positive PD-L1 expression. Therefore, there is an urgent need for a large randomized and controlled study to validate the therapeutic efficacy of dual immunotherapeutic drugs in combination with chemotherapy on populations with PDL1-expression-negative, driver-gene-negative, locally advanced or advanced metastatic NSCLC.

ZPML265 is a pharmaceutical formulation of a mixture of antibodies composed of a recombinant humanized IgG1 monoclonal antibody targeting human CTLA4 and a recombinant humanized IgG4 monoclonal antibody targeting human PD1, the two different antibodies being produced by a single host cell. The mixture of antibodies specifically binds to CTLA4 and PD1 at the same time, thereby blocking two immune checkpoint signaling pathways relating to CTLA4 and B7-1/B7-2 as well as PD-1 and PD-L1, relieving the inhibitory effects of the two pathways on T lymphocytes, restoring their functional activity and anti-tumor immune response, which in turn achieve the goal of combatting and killing tumor in the body.

Therefore, there is an unmet clinical need among patients with stage II-IIIB non-small cell lung cancer after complete surgical resection and PDL1-expression-negative, driver-gene-negative, locally advanced or metastatic non-small cell lung cancer. There is an urgent need to develop more effective medicines.

### Summary

The technical problem to be solved by the present disclosure is to provide an immunotherapy to fill the clinical gap of no dual-target immunotherapy drugs for non-small cell lung cancer in China, thus addressing this unmet clinical need.

The present disclosure provides a pharmaceutical composition comprising an effective amount of a mixture of antibodies of anti-CTLA4 and anti-PD1 for treating non-small cell lung cancer.

Meanwhile, the present disclosure also provides a method for treating non-small cell lung cancer using a pharmaceutical composition comprising an effective amount of a mixture of antibodies against CTLA4 and PD1.

In some embodiments, the pharmaceutical composition further comprises a pharmaceutically acceptable carrier.

In some embodiments, the pharmaceutical composition further comprises an additional therapeutic agent, which includes, but is not limited to, a chemotherapeutic agent (chemotherapeutic drug), a cytotoxic agent, a radiotherapeutic agent, a cancer vaccine, a cytoreductive agent, a targeted anticancer agent, an anti-angiogenic agent, a biological response modifier, a cytokine, a hormone, an anti-metastatic agent, and an immunotherapeutic agent.

In some embodiments, the additional therapeutic agent is a chemotherapeutic drug. Preferred chemotherapeutic drug is carboplatin/cisplatin, paclitaxel/vinorelbine, and/or pemetrexed/vinorelbine, wherein carboplatin and cisplatin may be used interchangeably and alternatively, paclitaxel and vinorelbine may be used interchangeably and alternatively, and pemetrexed and vinorelbine may be used interchangeably and alternatively.

In some embodiments, the non-small cell lung cancer described in the present disclosure is at stage III or IV according to the TNM staging.

In some other embodiments, the non-small cell lung cancer is non-small cell lung cancer at stage II-IIIB after complete surgical resection.

In some other embodiments, the non-small cell lung cancer is locally advanced or metastatic non-small cell lung cancer that is negative for PD-L1 expression, has no epidermal growth factor receptor (EGFR)-sensitive mutation or altered anaplastic lymphoma kinase (ALK) gene translocation, and has not been subjected to a systemic anti-tumor therapy.

In the present disclosure, the non-small cell lung cancer comprises squamous non-small cell lung cancer and non-squamous non-small cell lung cancer.

Preferably, when the non-small cell lung cancer is squamous non-small cell lung cancer, the mixture of antibodies is administered at a dose of 5 mg/kg by intravenous infusion on day 1 of each cycle, with one treatment cycle lasting 21 days. In addition, additional chemotherapeutic drugs, which are carboplatin/cisplatin and paclitaxel/vinorelbine, may be administered. Carboplatin is at a dose of 5mg/mL/min based on an area under the curve (AUC), or cisplatin is at a dose of 75mg/m²; and paclitaxel is at a dose of 175mg/m², or vinorelbine is at a dose of 25mg/m²; administration is performed by intravenous infusion on day 1 of each cycle, with one treatment cycle lasting 21 days; and if vinorelbine is selected, vinorelbine is administered at 25mg/m² again on day 8 of an administration cycle. Further, after the completion of 2 to 4 administration cycles of the mixture of antibodies and the chemotherapeutic drugs, the mixture of antibodies is used for maintenance treatment at a dose of 5 mg/kg once every three weeks by intravenous infusion.

Preferably, when the non-small cell lung cancer is non-squamous non-small cell lung cancer, the mixture of antibodies is administered at a dose of 5 mg/kg by intravenous infusion on day 1 of each cycle, with one treatment cycle lasting 21 days. In addition, additional chemotherapeutic drugs, which are carboplatin/cisplatin and pemetrexed/vinorelbine, may be administered. Carboplatin is at a dose of 5mg/mL/min based on an area under the curve (AUC), or cisplatin is at a dose of 75mg/m²; and pemetrexed is at a dose of 500mg/m², or vinorelbine is at a dose of 25mg/m²; administration is performed by intravenous infusion on day 1 of each cycle, with one treatment cycle lasting 21 days; and if vinorelbine is selected, vinorelbine is administered at 25mg/m² again on day 8 of an administration cycle. Further, after the completion of 2 to 4 administration cycles of the mixture of antibodies and the chemotherapeutic drugs, the mixture of antibodies is used for maintenance treatment at a dose of 5 mg/kg once every three weeks by intravenous infusion.

In some embodiments, the mixture of antibodies is produced by a single host cell comprising nucleic acids encoding two different antibodies, i.e. both a nucleic acid encoding an anti-CTLA4 antibody and a nucleic acid encoding an anti-PD1 antibody , wherein the sequences of heavy chain HCDR1, HCDR2 and HCDR3 of the anti-CTLA4 antibody are set forth in SEQ ID NOs:1, 2 and 3, respectively; the sequences of light chain LCDR1, LCDR2 and LCDR3 of the anti-CTLA4 antibody are set forth in SEQ ID NOs:4, 5 and 6, respectively; the sequences of heavy chain HCDR1, HCDR2 and HCDR3 of the anti-PD1 antibody are set forth in SEQ ID NOs:9, 10 and 11, respectively; and the sequences of light chain LCDR1, LCDR2 and LCDR3 of the anti-PD1 antibody are set forth in SEQ ID NOs: 12, 13 and 14, respectively.

In some embodiments, the sequence of heavy chain variable region of the anti-CTLA4 antibody is set forth in SEQ ID NO:7, the sequence of light chain variable region of the anti-CTLA4 antibody is set forth in SEQ ID NO:8, the sequence of heavy chain variable region of the anti-PD1 antibody is set forth in SEQ ID NO:15, and the sequence of light chain variable region of the anti-PD1 antibody is set forth in SEQ ID NO: 16.

In some embodiments, the sequence of heavy chain of the anti-CTLA4 antibody is set forth in SEQ ID NO: 17, the sequence of light chain of the anti-CTLA4 antibody is set forth in SEQ ID NO:18, the sequence of heavy chain of the anti-PD1 antibody is set forth in SEQ ID NO:19, and the sequence of light chain of the anti-PD1 antibody is set forth in SEQ ID NO:20.

Meanwhile, the present disclosure also provides the use of the mixture of antibodies comprising an effective amount of anti-CTLA4 antibody and anti-PD1 antibody and a chemotherapeutic drug in the manufacture of a pharmaceutical composition for treating stage II-IIIB non-small cell lung cancer after complete surgical resection.

In addition, the present disclosure also provides the use of the mixture of antibodies comprising an effective amount of anti-CTLA4 antibody and anti-PD1 antibody and a chemotherapeutic drug in the manufacture of a pharmaceutical composition for treating locally advanced or metastatic non-small cell lung cancer that is negative for PD-L1 expression, has no epidermal growth factor receptor (EGFR)-sensitive mutation or altered anaplastic lymphoma kinase (ALK) gene translocation, and has not been subjected to a systemic anti-tumor therapy.

Results from a phase I clinical study of the mixture of antibodies of the present disclosure as a monotherapy have shown that there are exact curative effects and adequate safety on the enrolled patients with non-small cell lung cancer. Further, clinical studies that have been conducted based on the mixture of antibodies of the present disclosure in combination with chemotherapy in non-small cell lung cancer have also shown a synergistic anti-tumor effect, exhibiting superior clinical efficacy. Therefore, the mixture of antibodies as a monotherapy, or a combined regimen of the mixture of antibodies + carboplatin/cisplatin + paclitaxel/vinorelbine, or of the mixture of antibodies + carboplatin/cisplatin + pemetrexed/vinorelbine provided in the present disclosure can be used for medical treatment of stage III or IV non-small cell lung cancer, is expected to be controllable in safety and to have good patient compliance, and can largely fill the blank where there is no dual-target immunotherapy drug approved for non-small cell lung cancer in China, thereby solving the unmet urgent clinical need.

### Detailed Description

### Terms

All publications, patents, and patent applications mentioned in this specification are incorporated herein by reference to the same extent as if each individual publication, patent, or patent application had been specifically and individually indicated to be incorporated by reference.

Before the present disclosure is described in detail below, it is to be understood that the present disclosure is not limited to the specific methodologies, protocols, and reagents described herein, as these may vary. It is also to be understood that the terms used herein are for the purpose of describing particular embodiments only and are not intended to limit the scope of the present disclosure. Unless defined otherwise, all technical and scientific terms used herein have the same meaning as commonly understood by one of ordinary skill in the art to which the present disclosure pertains.

Certain embodiments disclosed herein include numerical ranges, and certain aspects of the present disclosure may be described in terms of ranges. Unless otherwise indicated, it is to be understood that the numerical ranges or the manners described in terms of ranges are for purposes of brevity and convenience only, and are not to be considered as a strict limitation on the scope of the present disclosure. Accordingly, the description in terms of ranges should be considered to specifically disclose all possible sub-ranges and all possible specific numerical points within that range, as if such sub-ranges and numerical points had been expressly written herein. The above principles apply equally regardless of the width of the numerical values. When description is made by using a range, the range includes the endpoints of the range.

In the cases relating to a measurable value, such as an amount and a temporary time duration, the term "about" refers to a change by ±20%, in certain cases by ±10%, in certain cases by ± 5%, in certain cases ± by 1%, or in certain cases ± by 0.1%, of a specified value.

As used herein, three-letter codes and single-letter codes for amino acids are described in J. Biol. Chem, 243, p3558 (1968).

As used herein, the term "antibody" typically refers to a Y-shaped tetrameric protein containing two heavy (H) polypeptide chains (HC) and two light (L) polypeptide chains (LC) held together by covalent disulfide bonds and non-covalent interactions. A natural IgG antibody has such structure. Each light chain contains a light chain variable domain (VL) and a light chain constant domain (CL). Each heavy chain contains a heavy chain variable domain (VH) and a heavy chain constant domain (CH) (also known as heavy chain constant region (CH)).

Five major classes of antibodies are known in the art: IgA, IgD, IgE, IgG and IgM, their corresponding heavy chain constant domains being referred to as α, δ, ε, γ and µ, respectively. IgG and IgA can be further divided into different subclasses. For example, IgG can be divided into IgG1, IgG2, IgG3 and IgG4; and IgA can be divided into IgA1 and IgA2. A light chain of an antibody from any vertebrate species may be assigned to one of two apparently distinct types, termed κ and λ, based on the amino acid sequence of its constant domain.

The term "variable region" or "variable domain" presents significant changes in amino acid composition from one antibody to another and is primarily responsible for antigen recognition and binding. The variable regions of each light/heavy chain pair form an antigen binding site such that an intact IgG antibody has two binding sites (i.e., it is bivalent). The variable region (VH) of the heavy chain and the variable region (VL) of the light chain each contain three regions with extreme variability, referred to as hypervariable regions (HVR), or more generally, as complementarity determining regions (CDRs). VH and VL each having four backbone regions, FRs (or framework regions), denoted by FR1, FR2, FR3 and FR4, respectively. Thus, CDR and FR sequences typically occur in the following sequence of the heavy chain variable domain (VH) (or light chain variable domain (VL)): FR1-HCDR1 (LCDR1)-FR2-HCDR2 (LCDR2) -FR3-HCDR3 (LCDR3)-FR4.

Types of "antibodies" in a broad sense may include, for example, polyclonal antibodies, monoclonal antibodies, chimeric antibodies, humanized and primatized antibodies, CDR-grafted antibodies, human antibodies (including recombinantly produced human antibodies), recombinantly produced antibodies, intracellular antibodies, multispecific antibodies, bispecific antibodies, monovalent antibodies, multivalent antibodies, anti-idiotype antibodies, synthetic antibodies (including muteins and variants thereof), and the like.

The term "monoclonal antibody" refers to an antibody that is produced by a single cell clone and is substantially homogeneous, targeting only one particular epitope. Monoclonal antibody can be prepared using a variety of techniques known in the art, including hybridoma techniques, recombination techniques, phage display techniques, those with transgenic animals, synthetic techniques, or combinations of the foregoing, and the like.

It should be noted that the division of the CDRs and FRs of an antibody variable region of the present disclosure is determined according to the Kabat definition. Other naming and numbering systems, such as Chothia, IMGT or AHo, are also known to those skilled in the art. Thus, humanized antibodies comprising one or more CDRs derived from any naming system, based on the antibody sequences of the present disclosure, are expressly maintained within the scope of the present disclosure.

The term "antigen" refers to a substance that is recognized and specifically bound by an antibody or antibody binding fragment. In a broad sense, an antigen can include any immunogenic fragment or determinant of a selected target, including a single epitope, multiple epitopes, single domain, multiple domains, or intact extracellular domain (ECD) or protein.

The term "epitope" refers to a site on an antigen that specifically binds to an immunoglobulin or antibody. The epitope may be formed by adjacent amino acids, or non-adjacent amino acids juxtaposed due to tertiary folding of a protein.

The terms "polypeptide", "peptide", and "protein" are used interchangeably herein to refer to a polymer of amino acids of any length. The polymer may be linear, cyclic or branched. It may contain a modified amino acid, in particular a conservatively modified amino acid, and it may be interrupted by a non-amino acid. The terms also include, for example, an amino acid polymer that has been modified by glycosylation, lipidation, acetylation, phosphorylation, methylation, and the like.

As used herein, the term "mixture of antibodies" refers to that containing a limited number, optionally no more than two, three, four, five, six, seven, eight, nine or ten major antibody species produced from a host cell (optionally a cell from a single host cell line) that has been transfected with DNAs encoding at least two different antibodies (optionally full-length primate IgG antibodies) having different binding specificities. In some embodiments, DNAs encoding at least two different heavy chains (HC) and at least two different light chains (LC) may be introduced into the same host cell, for example, the host cell may be transfected with DNAs encoding at least two but no more than four different antibodies with different binding specificities. In some embodiments, the sequences of all transfected DNAs encoding HC and LC may be mutated, thereby altering the amino acid sequence of the antibody such that non-homologous HC/LC pairing is not facilitated, while homologous HC/LC pairing is highly facilitated. Where two different HCs are introduced into a host cell, one or both of the two different HCs may optionally be altered so that the formation of heterodimers is not facilitated. In some embodiments, only one heavy chain is altered to prevent the formation of heterodimers. In some embodiments, where DNAs encoding only two different antibodies are introduced into a host cell, only one of the antibodies encoded by the DNAs comprises one or more partner-directed alterations such that homologous HC/LC pairing is facilitated, while the other antibody does not comprise such alterations. In some embodiments, the host cell produces only two major antibody species, where each HC primarily paired with its homologous LC, and most antibodies are tetramers containing two heavy chains having the same amino acid sequence and two light chains having the same amino acid sequence (see PCT/US2017/030676).

The term "pharmaceutical composition" refers to a formulation or combination of formulations that comprises one, two or more active ingredients, allows the active ingredients contained therein to exist in a biologically effective form, and does not include additional ingredients with unacceptable toxicity to a subject to whom the formulation is administered. When the "pharmaceutical composition" is in the form of a combination of separate formulations containing two or more different active ingredients, they may be administered simultaneously, sequentially, separately or at intervals, the purpose of which is to exert biological activities of the various active ingredients, together for the treatment of the disease(s).

The term "pharmaceutical carrier" or "pharmaceutically acceptable carrier" refers to a diluent, an adjuvant (e.g., Freund's adjuvant (complete and incomplete)), an excipient, or a vehicle administered with a therapeutic agent.

The term "effective amount" refers to a dosage of a pharmaceutical formulation comprising the active ingredients of the present disclosure that, upon administration to a patient in a single dose or multiple doses, produces desired effects in the patient being treated. An effective amount can be easily determined by an attending physician as a person skilled in the art by considering a variety of factors such as race differences; weight, age, and health status; specific disease involved; severity of the disease; response of an individual patient; specific antibodies administered; administration mode; bioavailability profile of the administrated formulation; selected dosing regimen; and use of any concomitant therapy.

The terms "host cell", "host cell line" and "host cell culture" may be used interchangeably and refer to a cell with an exogenous nucleic acid introduced therein, including a progeny of such cell. Host cell includes a "transformant" and a "transformed cell", which includes an initially transformed cell and a progeny originated therefrom, regardless of the number of passages. The progeny may not be identical in nucleic acid content to the parent cell and may contain mutation(s). Included herein are mutated progenies having the same function or biological activity as those screened or selected from the initially transformed cell.

As used herein, the term "transfection" refers to the introduction of an exogenous nucleic acid into a eukaryotic cell. Transfection can be accomplished by a variety of means known in the art, including calcium phosphate-DNA co-precipitation, DEAE-dextran-mediated transfection, polybrene-mediated transfection, electroporation, microinjection, liposome fusion, lipid transfection, protoplast fusion, retroviral infection, and biolistics.

The term "isolated polynucleotide" or "isolated nucleic acid" refers to a nucleic acid molecule, DNA or RNA that has been removed from its native environment. For example, for the purposes of the present disclosure, a recombinant polynucleotide encoding a polypeptide contained in a vector is considered isolated. Other examples of an isolated polynucleotide include a recombinant polynucleotide maintained in a heterologous host cell or a (partially or substantially) purified polynucleotide in a solution. An isolated polynucleotide includes a polynucleotide molecule contained in a cell typically containing the polynucleotide molecule, but the polynucleotide molecule is present outside the chromosome or at a chromosomal location different from its native chromosomal location. An isolated RNA molecule includes an in vivo or in vitro RNA transcript of the present disclosure, as well as its positive- and negative-stranded forms and double-stranded forms. An isolated polynucleotide or nucleic acid of the present disclosure also includes such molecule produced synthetically. In addition, the polynucleotide or nucleic acid may or may not include a regulatory element such as a promoter, a ribosome binding site, or a transcription terminator.

The terms "nucleic acid molecule code", "encoding DNA sequence" and "encoding DNA" refer to the order of deoxyribonucleotides along the deoxyribonucleic acid chain. The order of these deoxyribonucleotides determines the order of the amino acids along the polypeptide (protein) chain. Thus, the nucleic acid sequence encodes an amino acid sequence.

Methods for producing and purifying an antibody and an antigen-binding fragment are well known and can be found in the prior art, such as in the Antibodies: A Laboratory Manual, Chapters 5-8 and 15, by Cold Spring Harbor. The engineered antibodies or antigen-binding fragments thereof in the present disclosure can be prepared and purified by conventional methods. For example, cDNA sequences encoding heavy and light chains may be cloned and recombined into an expression vector. The recombinant vector for immunoglobulin expression may be stably transfected into CHO cells. A stable clone is obtained by expressing antibodies that specifically bind to human antigens. The positive clone is expanded in a serum-free medium in a bioreactor to produce the antibodies. The culture medium into which the antibodies are secreted may be purified and collected by conventional techniques. The antibodies may be filtrated and concentrated using conventional methods. Soluble mixtures and multimers may also be removed by conventional methods, such as molecular sieves and ion exchange.

As used herein, the term "individual" or "subject" refers to any animal, such as a mammal or a marsupial. The individual of the present disclosure includes, but is not limited to, a human, a non-human primate (e.g., a cynomolgus monkey, a rhesus monkey or a macaque monkey of another type), a mouse, a pig, a horse, a donkey, a bovine, a sheep, a rat, and a poultry of any kind.

As used herein, the term "disease", "condition", "disorder" or the like refers to any alteration or dysregulation that impairs or interferes with the normal function of a cell, tissue or organ. For example, the "disease" includes, but is not limited to, a tumor, an infection with a pathogen, an autoimmune disease, a T-cell-dysfunctional disease, or deficiency in immunotolerance (e.g., graft rejection), and the like.

As used herein, the term "tumor" refers to a disease characterized by pathological proliferation of cells or tissues, and their subsequent migration or invasion to other tissues or organs. Tumor growth is generally uncontrolled and progressive, and does not induce or inhibit normal cell proliferation. Tumor includes "cancer", which refers generally to all malignancies.

As used herein, the term "treatment" refers to a clinical intervention in attempting to alter a person or treat a disease caused by a cell, either prophylactically or in a clinically pathological process. Therapeutic effects include, but are not limited to, prevention of the onset or recurrence of a disease, alleviation of symptoms, reduction of direct or indirect pathological consequences of any disease, prevention of metastasis, slowing of the rate of progression of a disease, amelioration or alleviation of a disease state, alleviation or improvement of prognosis, and the like.

As used herein, the term "in combination with" involves a treatment regimen that provides at least two or more than two different therapies that may be physical, such as radiation therapy, or may be chemical, e.g. administering to a subject a medicine that also includes medicines for combination use, to achieve a specified therapeutic effect. "Medicines for combination use" refers to a combination comprising two or more pharmaceutical formulations each having an active ingredient, which need to be used in combination when administered to a subject. The active ingredients may be mixed together to form a single administration unit, or may be separately used as individual administration units, respectively. At the time of administration, the different pharmaceutical formulations may be administered substantially synchronously, simultaneously or sequentially.

Overall survival (OS) refers to the period between the initiation of study treatment and the subject's death from any cause. Patients who were still at follow-up as of the analysis cutoff date (Cut-off) were censored at the analysis cutoff date, and subjects who were lost to follow-up were censored at the date of their last contact.

Progression-free survival (PFS) is the period between the initiation of study treatment and the subject's first imaging assessment of disease progression or death of any cause (whichever occurs first).

Objective response rate (ORR) is defined as the best ORR confirmed during the study, including cases of complete response (CR) and partial response (PR). According to RECIST vl.l, when PR or CR is first present, confirmation needs to be made by additional imaging examination of lesions at ≥4 weeks.

The disease control rate (DCR) refers to the percentage of cases with an optimal efficacy evaluation of "CR+PR+SD".

Complete response (CR): all target lesions disappear and the short diameters of all pathological lymph nodes (including target and non-target nodules) must be reduced to <10mm.

The partial response (PR): the sum of the diameters of target lesions is reduced by at least 30% from the baseline level.

Disease progression (PD): a relative increase by at least 20% in the sum of the diameters of all target lesions measured throughout the course of the experimental study with reference to the minimum of the sum of the diameters of all target lesions measured (with reference to the baseline value if the baseline measurement is the minimum); and otherwise the absolute value of the sum of the diameters must be increased by at least 5 mm (the appearance of one or more new lesions is also considered to be disease progression).

Stable disease (SD): the degree of reduction of a target lesion does not reach PR, the degree of its increase does not reach a PD level, and it is between the two, where the minimum value of the sum of the diameters can be used as a reference for the study.

Duration of response (DOR) is defined as the period between the first assessment of a tumor as CR or PR (whichever is recorded first) and the first assessment as PD or death.

A treatment emergent adverse event (TEAE) refers to an adverse event occurring during the treatment.

A treatment-related adverse event (TRAE) refers to an adverse event associated with a study drug occurring during the treatment.

A serious adverse event (SAE) is an adverse medical event (AE), such as death, life-threatening, permanent or serious disability or loss of function, needs for hospitalization or prolongation of hospitalization time, and a congenital abnormality or birth defect, which occurs after a subject receives an investigational drug.

### Examples

The present disclosure will be further illustrated below in connection with specific Examples. It should be understood that these Examples are merely illustrative of the present disclosure and are not intended to limit the scope of the present disclosure.

### Example 1 Obtaining the mixture of antibodies ZPML265

A mixture of antibodies was produced by a single host cell line using the method disclosed in PCT/US2017/030676, and the mixture of antibodies contains two active ingredients, a recombinant humanized IgG1 monoclonal antibody targeting human CTLA4 and a recombinant humanized IgG4 monoclonal antibody targeting human PD1. The mixture of antibodies can specifically bind to both of human CTLA4 and PD1. Both antibodies were expressed simultaneously by the single host cell line, collected, purified, and combined with a pharmaceutically acceptable carrier to form a single pharmaceutical preparation of the mixture of antibodies, ZPML265. The amino acid sequences of each of the anti-CTLA4 antibody and the anti-PD1 antibody are shown in Table 1 below.

**Table 1 Amino acid sequence of each component of the mixture of antibodies ZPML265**

| Name | SEQ ID NO: |
|---|---|
| Heavy chain HCDR1 of anti-CTLA4 antibody | 1 |
| Heavy chain HCDR2 of anti-CTLA4 antibody | 2 |
| Heavy chain HCDR3 of anti-CTLA4 antibody | 3 |
| Light chain LCDR1 of anti-CTLA4 antibody | 4 |
| Light chain LCDR2 of anti-CTLA4 antibody | 5 |
| Light chain LCDR3 of anti-CTLA4 antibody | 6 |
| Heavy chain variable region VH of anti-CTLA4 antibody | 7 |
| Light chain variable region VL of anti-CTLA4 antibody | 8 |
| Heavy chain HCDR1 of anti-PD1 antibody | 9 |
| Heavy chain HCDR2 of anti-PD1 antibody | 10 |
| Heavy chain HCDR3 of anti-PD1 antibody | 11 |
| Light chain LCDR1 of anti-PD1 antibody | 12 |
| Light chain LCDR2 of anti-PD1 antibody | 13 |
| Light chain LCDR3 of anti-PD1 antibody | 14 |
| Heavy chain variable region VH of anti-PD1 antibody | 15 |
| Light chain variable region VL of anti-PD1 antibody | 16 |
| Heavy chain HC of anti-CTLA4 antibody | 17 |
| Light chain LC of anti-CTLA4 antibody | 18 |
| Heavy chain HC of anti-PD1 Antibody | 19 |
| Light chain LC of anti-PD1 antibody | 20 |

### Example 2 A clinical study to evaluate the safety and effectiveness of ZPML265 in combination with chemotherapy in first-line treatment of patients with non-small cell lung cancer

In a phase II clinical study conducted in China, a total of 29 patients with squamous cell carcinoma and non-squamous cell carcinoma without mutations in EGFR/ALK/ROS1 genes were enrolled. The patients were aged ≥18 years, with American Eastern Cooperative Oncology Group (ECOG) performance status scores of 0 or 1, expected survival time of ≥3 months, and at least 1 measurable lesion according to RECIST vl.l. The functional levels of vital organs before the first administration of the investigational drug were as follows:
1) leukocytes ≥ 3 × 10⁹/L;
2) absolute neutrophil count ≥ 1.5 × 10⁹/L;
3) platelets ≥ 100× 10⁹/L;
4) hemoglobin ≥ 90g/L;
5) serum creatinine ≤ 1.5×upper limit of normal (ULN) or creatinine clearance (CLcr) calculated by Cockcroft-Gault formula ≥ 50mL/min;
6) total bilirubin ≤ 1.5×ULN (patients with Gilbert syndrome may be < 3×ULN);
7) aspartate aminotransferase (AST) and alanine transaminase (ALT) ≤ 2.5×ULN (where there were liver metastases, ≤ 5×ULN was permissible); and
8) left ventricular ejection fraction (LVEF) > 50%.

### Dosing Regimen

Medicines were used in this study as follows. The treatment received by the patients was divided into two phases, the lead-in period and the post-lead-in period, respectively, with the lead-in period including two cycles. ZPML265 was administered in combination with chemotherapy for 2 cycles during the lead-in period for squamous cell carcinoma and non-squamous cell carcinoma, followed by maintenance treatment with ZPML265. ZPML265 was administered until disease progression, intolerable toxicity, initiation of other antitumor therapies, loss to follow-up, death, withdrawal from the study, treatment for up to 2 years and the like, whichever occurs first. The dosing window was ±3 days from the scheduled dosing date (calculated from the date after the first dosing). If the dosing window was exceeded, the dosing was considered to be in advance or delayed, and the subsequent dosing dates need to be recalculated according to the last actual dosing date.

ZPML265: 5 mg/kg, administered once every three weeks (Q3W), with intravenous infusion (IV) on day 1. ZPML265 was intravenously infused for more than 30 min. After the completion of the infusion, 20 ml of the same solvent was used for tube flushing. The entire infusion time was 60 min (±5min) with the time for tube flushing being included. Intravenous bolus injection of ZPML265 was not permissible.

Chemotherapy regimen:
Patients with non-squamous cell carcinoma: pemetrexed (500mg/m²) + carboplatin (AUC 5 mg/mL/min or 6 mg/mL/min) (according to the Calvert formula), administered once every three weeks with intravenous fusion on day 1 of each cycle. Administration was performed for two cycles.

Patients with squamous cell carcinoma: paclitaxel (175mg/m²) + carboplatin (AUC 5 mg/mL/min or 6 mg/mL/min) (according to the Calvert formula), administered once every three weeks with intravenous infusion on day 1 of each cycle. Administration was performed for two cycles.

### Results for effectiveness

Efficacy data for the full analysis set: 58.6% (17/29) for ORR and 93.1% (27/29) for DCR.

Analysis was performed according to pathologic classification (squamous and non-squamous):
(1) 17 patients with wild-type squamous NSCLC included for first-line treatment with ZPML265 (5 mg/kg) + paclitaxel (175mg/m²) + carboplatin (AUC5/6), with 71% (12/17) for ORR and 94% (16/17) for DCR.
(2) 12 patients with wild-type non-squamous NSCLC included for first-line treatment with ZPML265 (5 mg/kg) + pemetrexed (500mg/m²) + carboplatin (AUC5/6), with 42% (5/12) for ORR and 92% (11/12) for DCR.

The above results demonstrated that the treatment regimen of ZPML265 (5 mg/kg) in combination with chemotherapy was effective for both squamous and non-squamous cell carcinomas.

### Results for safety

All 29 patients with evaluable safety experienced TRAEs. The incidence of grade ≥3 AEs was 13.8% (4/29), of which 2 cases (6.9%) were associated with ZPML265. The incidence of infusion-related reactions was 67.0% (20/29). The incidence of immune-related adverse events was 75.9% (22/29). The incidence of SAEs was 24.1% (7/29). The incidence of study drug-related SAEs was 17.2% (5/29). The incidence of AEs leading to study drug discontinuation was 41.4% (12/29). No AEs leading to permanent discontinuation of the study drug occurred.

### Preliminary conclusions

ZPML265 in combination with chemotherapy showed a significant efficacy trend, as well as controllable overall safety and good tolerance, in patients with stage IIIB/C and stage IV non-small cell lung cancer.

### Example 3 A phase II clinical study to evaluate the safety and effectiveness of ZPML265 in combination with chemotherapy and bevacizumab in first-line treatment of patients with non-small cell lung cancer

In a phase II clinical study conducted in China, a total of 31 patients with stage IIIB/C and stage IV wild-type EGFR non-small cell lung cancer (including squamous and non-squamous cell carcinomas) and 31 patients with non-small cell lung cancer who failed treatment with EGFR-TKI were enrolled.

The patients were required to be aged above 18 years, with American Eastern Cooperative Oncology Group (ECOG) performance status scores of 0 or 1, expected survival time of ≥3 months, and at least 1 measurable lesion according to RECIST vl.l. The functional levels of vital organs before the first administration of the investigational drug were as follows:
1) leukocytes ≥ 3 × 10⁹/L;
2) absolute neutrophil count ≥ 1.5 × 10⁹/L;
3) platelets ≥ 100× 10⁹/L;
4) hemoglobin ≥ 90g/L;
5) serum creatinine ≤ 1.5×upper limit of normal (ULN) or creatinine clearance (CLcr) calculated by Cockcroft-Gault formula ≥ 50mL/min;
6) total bilirubin ≤ 1.5×ULN (patients with Gilbert syndrome may be < 3×ULN);
7) AST and ALT ≤ 2.5 ×ULN (where there were liver metastases, ≤ 5×ULN was permissible);
8) INR or APTT ≤ 1.5×ULN; and
9) left ventricular ejection fraction (LVEF) > 50%.

### Dosing Regimen

Medicines were used in this study as follows. The treatment received by the patients was divided into two phases, the lead-in period and the post-lead-in period, respectively, with the lead-in period including 4 cycles. ZPML265 was administered in combination with chemotherapy and bevacizumab for 4 cycles during the lead-in period for non-squamous cell carcinoma, followed by maintenance treatment with ZPML265 in combination with pemetrexed and bevacizumab. ZPML265 was administered in combination with chemotherapy for 4 cycles during the lead-in period for squamous cell carcinoma, followed by maintenance treatment with ZPML265. ZPML265 was administered until disease progression, intolerable toxicity, initiation of other antitumor therapies, loss to follow-up, death, withdrawal from the study, treatment for up to 2 years and the like, whichever occurs first. The dosing window was ±3 days from the scheduled dosing date (calculated from the date after the first dosing). If the dosing window was exceeded, the dosing was considered to be in advance or delayed, and the subsequent dosing dates need to be recalculated according to the last actual dosing date.

ZPML265: 5 mg/kg, administered once every three weeks, with intravenous infusion on day 1. The time for the intravenous infusion of ZPML265 was more than 30 min. After the completion of the infusion, 20 ml of the same solvent was used for tube flushing. The entire infusion time was 60 min (±5min) with the time for tube flushing being included. Intravenous bolus injection of ZPML265 was not permissible.

Chemotherapy regimen:
Patients with non-squamous cell carcinoma: during the lead-in period, ZPNEL265 (5 mg/kg) + pemetrexed (500mg/m²) + carboplatin (AUC5/6) + bevacizumab (15 mg/kg) administered once every three weeks with intravenous infusion on day 1, for 4 cycles; during the post-lead-in period, ZPML265 (5 mg/kg) + pemetrexed (500mg/m²) + bevacizumab (15 mg/kg) administered once every three weeks with intravenous infusion on day 1.

Patients with non-squamous cell carcinoma: during the lead-in period, ZPML265 (5 mg/kg) + paclitaxel (175mg/m²) + carboplatin (AUC5/6) administered once every three weeks with intravenous infusion on day 1, for 4 cycles; during the post-lead-in period, ZPML265 (5 mg/kg) administered once every three weeks with intravenous infusion on day 1.

### Results for effectiveness (data for wild-type EGFR NSCLC)

### (1) Effectiveness in the whole population

There were 31 cases of wild-type EGFR NSCLC in total, with 45.2% (14/31) for ORR, 83.9% (26/31) for DCR, and 5.45 (4.271, 11.236) for PFS.

Analysis was performed according to pathologic classification (squamous and non-squamous):
(1) 11 patients with wild-type squamous NSCLC included for first-line treatment with ZPML265 (5 mg/kg) + paclitaxel (175mg/m²) + carboplatin (AUC5/6) for 4 cycles, with 36.4% (4/11) for ORR, 90.9% (10/11) for DCR, and with mPFS of 6.97 (4.107, -) months.
(2) 20 patients with wild-type non-squamous NSCLC included for first-line treatment with ZPML265 (5 mg/kg) + pemetrexed (500mg/m²) + carboplatin (AUC5/6) + bevacizumab (15 mg/kg) for 4 cycles, with 40% (8/20) for ORR, 94.1% (16/20) for DCR, and with mPFS of 7.69 (4.271, -) months.

The above results demonstrated that the treatment regimen of ZPML265 (5 mg/kg) in combination with chemotherapy was effective for both squamous and non-squamous cell carcinomas.

### (2) Subjects with negative PD-L1 expression - preliminary effectiveness in the target study population

Taken Examples 2 and 3 together, a total of 12 NSCLC patients with negative PD-L1 expression and negative driver genes for first-line treatment were treated with ZPML265 in combination with platinum-containing chemotherapy ± bevacizumab, with an initial response rate of 50% (6/12), a DCR of 91.7% (11/12), and a mPFS of 6.14 (2.103, -) months, suggesting that the ZPML265 combination therapy had a good therapeutic potential in NSCLC patients with negative PD-L1 expression and negative driver genes for first-line treatment with a good response, and adverse effects are tolerable.

### Results for Safety

### The following are results for safety from a comprehensive analysis of Example 2 (wild type NSCLC, 29 cases) and Example 3 (wild type EGFR NSCLC, 31 cases).

Of the 60 patients with evaluable safety, 59 experienced TRAEs. The incidence of grade ≥3 AEs was 40% (24/60), of which 13 cases (21.7%) were associated with ZPML265, which was lower than that in similar drug studies (the incidence in the corresponding CM-9LA study was 47%, and the incidence in the POSEIDON study was 51.8%). 41 cases (68.3%) had immune-related adverse events, of which 35 cases (58.3%) were associated with ZPML265. 34 cases (56.7%) had TEAEs leading to drug discontinuation, of which 30 cases (50.0%) were associated with ZPML265. 3 cases (5.0%) had TEAEs leading to permanent drug discontinuation, of which 2 cases (3.3%) were associated with ZPML265. These values were lower than that in similar studies (the incidence in the corresponding CM-9LA study was 19%, and the incidence in the POSEIDON study was 15.1%), and there were no adverse effects leading to death in the study.

### Preliminary conclusions

The regime of ZPML265 in combination with chemotherapy showed a significant efficacy trend, as well as controllable overall safety and good tolerance, in patients with stage IIIB/C and stage IV non-small cell lung cancer (including NSCLC with negative drive genes and negative PD-L1 expression).

### Example 4 A phase III clinical study to evaluate the effectiveness and safety of ZPML265 in combination with chemotherapy in first-line treatment of patients with PD-L1-negative, locally advanced or metastatic non-small cell lung cancer

In a phase III clinical study conducted in China, 650 cases with locally advanced or metastatic non-small cell lung cancer with negative PD-L1 expression, including squamous and non-squamous carcinomas, were scheduled to be enrolled. NSCLC populations with mutations in EGFR and ALK were excluded.

Negative PD-L1 expression was defined as TPS<1% (TPS: percentage of tumor cells having PD-L1 staining of any intensity on their membrane among tumor cells).

The patients were required to be aged above 18 years, with American Eastern Cooperative Oncology Group (ECOG) performance status scores of 0 or 1, expected survival time of≥3 months, at least 1 measurable lesion according to RECIST vl.l, and no prior systemic treatment for advanced or metastatic NSCLC. The functional levels of vital organs before the first administration of the investigational drug were as follows:
(1) absolute neutrophil count ≥ 1.5 × 10⁹/L;
(2) platelet count ≥ 100×10⁹/L;
(3) hemoglobin ≥ 90g/L;
(4) serum creatinine ≤ 1.5×upper limit of normal (ULN) or creatinine clearance (CLcr) calculated by Cockcroft-Gault formula ≥ 50mL/min;
(5) total bilirubin ≤ 1.5×ULN (patients with Gilbert syndrome may be ≤ 3×ULN);
(6) AST and ALT ≤ 2.5×ULN (patients with liver metastases was ≤ 5×ULN);
(7) international normalized ratio (INR) or activated partial thromboplastin time (APTT) ≤1.5×ULN, unless the subject was receiving an anticoagulant therapy; and
8) left ventricular ejection fraction (LVEF) ≥ 50%.

### Dosing Regimen

Medicines were used in this study as follows. The treatment received by the subject was divided into 2 phases, the lead-in period and the maintenance period, respectively, with the lead-in period including 4 cycles. Subjects received the treatment regimen indicated in the following table by study grouping and tissue typing until disease progression, intolerable toxicity and side effects, withdrawal of consent, non-compliance with study treatment or study procedures, investigator's decision to withdraw the subjects from the study or other reasons specified in the regimen (maximum duration of ZPML265 treatment was 2 years [for 35 cycles]).

Table 2 Regime of the phase III clinical study to evaluate the effectiveness and safety of ZPML265 in combination with chemotherapy in the first-line treatment of patients with PD-L1-negative, locally advanced or metastatic non-small cell lung cancer

| Group | Pathological classification of NSCLC | Lead-in period (with 3 weeks as a cycle) | Maintenance period (with 3 weeks as a cycle) |
|---|---|---|---|
| Test group | Squamous cell carcinoma | ZPML265 5mg/kg Q3W (D1) + paclitaxel 175mg/m² Q3W (D1) + carboplatin (AUC=5) Q3W (D1) for 4 cycles of treatment | ZPML265 5mg/kg Q3W (D1) |
| | Non-squamous cell carcinoma | ZPML265 5mg/kg Q3W (D1) + pemetrexed 500mg/m² Q3W (D1) + carboplatin (AUC=5) Q3W (D1) for 4 cycles of treatment | ZPML265 5mg/kg Q3W (D1) + pemetrexed 500mg/m² Q3W (D1) |
| Control group | Squamous cell carcinoma | Tislelizumab 200mg Q3W (D1) + paclitaxel 175mg/m² Q3W (D1) + carboplatin (AUC=5) Q3W (D1) for 4 cycles of treatment | Tislelizumab 200mg Q3W (D1) |
| | Non-squamous cell carcinoma | Tislelizumab 200mg Q3W (D1) + pemetrexed 500mg/m² Q3W (D1) + carboplatin (AUC=5) Q3W (D1) for 4 cycles of treatment | Tislelizumab 200mg Q3W (D1) + pemetrexed 500mg/m² Q3W (D1) |

### Example 5 A randomized, double-blinded, multicenter phase III clinical study of ZPML265 in combination with platinum-containing chemotherapy versus placebo in combination with platinum-containing chemotherapy for adjuvant treatment of stage II-IIIB non-small cell lung cancer after complete surgical resection

In a randomized, double-blinded, multicenter phase III clinical study of ZPML265 in combination with platinum-containing chemotherapy versus placebo in combination with platinum-containing chemotherapy for adjuvant treatment of stage II-IIIB non-small cell lung cancer after complete surgical resection, patients with histopathologically confirmed squamous or non-squamous non-small cell lung cancer were enrolled. The patients were aged ≥ 18 years, unlimited for men and women, had ECOG performance status scores of 0-1, at stage II-IIIB according to the Eighth Edition of the American Joint Committee on Cancer (AJCC) staging system and had been treated with radical surgical resection (R0). The disease stages included T2b-T4N0, T1-T4N1, Tl-T2bN2, and resectable T3-T4N2. Types of resection procedures received included: pulmonary lobectomy, bronchial sleeve lobectomy, pulmonary bilobectomy, or pneumonectomy. Noted: segmental or wedge resection was not allowed. Patients should be enrolled for the adjuvant treatment within 8 weeks (≤56 days) after the surgery and had to recover sufficiently from the surgery to be able to receive investigational drug. No EGFR-sensitive mutation or ALK fusion gene (results from the detection by a local medical facility was accepted; and if not tested, tumor tissue specimens were provided for central laboratory testing prior to randomization) was in non-squamous NSCLC subjects. Sufficient organ functions (no blood components, leukocyte-raising medicines, platelet-raising medicines were allowed within 7 days before results of laboratory examination were obtained) prior to the first treatment by the investigational therapy.
1) leukocytes ≥ 4×10⁹/L;
2) absolute neutrophil count ≥ 1.5×10⁹/L;
3) platelet count ≥ 100×10⁹/L;
4) hemoglobin ≥ 90g/L;
5) serum creatinine ≤ 1.5×upper limit of normal (ULN) or creatinine clearance (CLcr) calculated by Cockcroft-Gault formula ≥ 50mL/min;
6) total bilirubin ≤ 1.5×ULN (patients with Gilbert syndrome may be ≤ 3×ULN);
7) AST and ALT ≤ 2.5×ULN;
8) INR or APTT ≤ 1.5×ULN; and
9) left ventricular ejection fraction (LVEF) ≥ 50%.

### Dosing Regimen

ZPML265: 5mg/kg, once every three weeks (Q3W), i.e. with three weeks as one dosing cycle, for intravenous infusion (IV) on day 1 (D1) of each cycle.

Placebo: a formulation for control group without the active ingredients in ZPML265, but with other excipients being the same as those in ZPML265, for intravenous infusion.

Chemotherapeutic drugs, including paclitaxel, pemetrexed, vinorelbine, cisplatin, carboplatin, for intravenous infusion.

### 1) Subjects with squamous NSCLC were randomized to receive one of the following treatments:

Test group: ZPML265 5mg/kg (D1) + vinorelbine 25mg/m² (D1, D8) or paclitaxel 175mg/m² (D1) + cisplatin 75mg/m² (D1) or carboplatin AUC=5mg/mL/min (D1), with three weeks as one dosing cycle, for 2-4 dosing cycles in total; followed by maintenance with ZPML265 5mg/kg (D1) dosed once every three weeks until 16 cycles of ZPML265 completed.

Control group: placebo 5 mg/kg (D1) + vinorelbine 25mg/m² (D1, D8) or paclitaxel 175mg/m² (D1) + cisplatin 75mg/m² (D1) or carboplatin AUC=5mg/mL/min (D1), with three weeks as one dosing cycle, for 2-4 dosing cycles in total; followed by maintenance with ZPML265 5mg/kg (D1) dosed once every three weeks until 16 cycles of placebo completed.

### 2) Subjects with non-squamous NSCLC were randomized to receive one of the following treatments:

Test group: ZPML265 5mg/kg (D1) + pemetrexed 500mg/m² (D1) or vinorelbine 25mg/m² (D1, D8) + cisplatin 75mg/m² (D1) or carboplatin AUC=5mg/mL/min (D1), with three weeks as one dosing cycle, for 2-4 dosing cycles in total; followed by maintenance with ZPML265 5mg/kg (D1) dosed once every three weeks until 16 cycles of ZPML265 completed.

Control group: placebo 5 mg/kg (D1) + pemetrexed 500mg/m² (D1) or vinorelbine 25mg/m² (D1, D8) + cisplatin 75mg/m² (D1) or carboplatin AUC=5mg/mL/min (D1), with three weeks as one dosing cycle, for 2-4 dosing cycles in total; followed by maintenance with ZPML265 5mg/kg (D1) dosed once every three weeks until 16 cycles of placebo completed.

## Claims

1. A pharmaceutical composition for treating non-small cell lung cancer, comprising an effective amount of a mixture of antibodies of anti-CTLA4 and anti-PD1, the mixture of antibodies being produced by a single host cell comprising both a nucleic acid encoding an anti-CTLA4 antibody and a nucleic acid encoding an anti-PD1 antibody, wherein the sequences of heavy chain HCDR1, HCDR2 and HCDR3 of the anti-CTLA4 antibody are set forth in SEQ ID NOs:1, 2 and 3, respectively; the sequences of light chain LCDR1, LCDR2 and LCDR3 of the anti-CTLA4 antibody are set forth in SEQ ID NOs:4, 5 and 6, respectively; the sequences of heavy chain HCDR1, HCDR2 and HCDR3 of the anti-PD1 antibody are set forth in SEQ ID NOs:9, 10 and 11, respectively; and the sequences of light chain LCDR1, LCDR2 and LCDR3 of the anti-PD1 antibody are set forth in SEQ ID NOs: 12, 13 and 14, respectively.

2. The pharmaceutical composition of claim 1, wherein the sequence of heavy chain variable region of the anti-CTLA4 antibody is set forth in SEQ ID NO:7, the sequence of light chain variable region of the anti-CTLA4 antibody is set forth in SEQ ID NO:8, the sequence of heavy chain variable region of the anti-PD1 antibody is set forth in SEQ ID NO: 15, and the sequence of light chain variable region of the anti-PD1 antibody is set forth in SEQ ID NO:16.

3. The pharmaceutical composition of claim 1, wherein the sequence of heavy chain of the anti-CTLA4 antibody is set forth in SEQ ID NO: 17, the sequence of light chain of the anti-CTLA4 antibody is set forth in SEQ ID NO:18, the sequence of heavy chain of the anti-PD1 antibody is set forth in SEQ ID NO: 19, and the sequence of light chain of the anti-PD1 antibody is set forth in SEQ ID NO:20.

4. The pharmaceutical composition of claim 1, wherein the non-small cell lung cancer is at stage III or stage IV according to the TNM staging.

5. The pharmaceutical composition of claim 1, wherein the non-small cell lung cancer is at stage II-IIIB after complete surgical resection.

6. The pharmaceutical composition of claim 1, wherein the non-small cell lung cancer is locally advanced or metastatic non-small cell lung cancer that is negative for PD-L1 expression, has no EGFR-sensitive mutation or altered ALK gene translocation, and has not been subjected to a systemic anti-tumor therapy.

7. The pharmaceutical composition of any one of claims 4-6, wherein the non-small cell lung cancer is squamous non-small cell lung cancer.

8. The pharmaceutical composition of claim 7, wherein the mixture of antibodies is administered at a dose of 5 mg/kg by intravenous infusion on day 1 of each cycle, with one treatment cycle of 21 days.

9. The pharmaceutical composition of claim 8, further comprising chemotherapeutic drugs.

10. The pharmaceutical composition of claim 9, wherein the chemotherapeutic drugs are carboplatin/cisplatin and paclitaxel/vinorelbine.

11. The pharmaceutical composition of claim 10, wherein carboplatin is at a dose of 5mg/mL/min based on an area under the curve (AUC), or cisplatin is at a dose of 75mg/m²; and paclitaxel is at a dose of 175mg/m², or vinorelbine is at a dose of 25mg/m²; administration is performed by intravenous infusion on day 1 of each cycle, with one treatment cycle of 21 days; and if vinorelbine is selected, vinorelbine is administered at 25mg/m² again on day 8 of an administration cycle.

12. The pharmaceutical composition of claim 11, wherein after the completion of 2 to 4 administration cycles of the mixture of antibodies and the chemotherapeutic drugs, the mixture of antibodies is used for maintenance treatment at a dose of 5 mg/kg once every three weeks by intravenous infusion.

13. The pharmaceutical composition of any one of claims 4-6, wherein the non-small cell lung cancer is non-squamous non-small cell lung cancer.

14. The pharmaceutical composition of claim 13, wherein the mixture of antibodies is administered at a dose of 5 mg/kg by intravenous infusion on day 1 of each cycle, with one treatment cycle of 21 days.

15. The pharmaceutical composition of claim 14, further comprising chemotherapeutic drugs.

16. The pharmaceutical composition of claim 15, wherein the chemotherapeutic drugs are carboplatin/cisplatin and pemetrexed/vinorelbine.

17. The pharmaceutical composition of claim 16, wherein carboplatin is at a dose of 5mg/mL/minbased on an area under the curve (AUC), or cisplatin is at a dose of 75mg/m²; and pemetrexed is at a dose of 500mg/m², or vinorelbine is at a dose of 25mg/m²; administration is performed by intravenous infusion on day 1 of each cycle, with one treatment cycle of 21 days; and if vinorelbine is selected, vinorelbine is administered at 25mg/m² again on day 8 of an administration cycle.

18. The pharmaceutical composition of claim 17, wherein after the completion of 2 to 4 administration cycles of the mixture of antibodies and the chemotherapeutic drugs, the mixture of antibodies is used for maintenance treatment at a dose of 5 mg/kg once every three weeks by intravenous infusion.

19. A method for treating non-small cell lung cancer comprising administering to a subject the pharmaceutical composition of any one of claims 1-18.

20. Use of a mixture of antibodies comprising an effective amount of anti-CTLA4 and anti-PD 1 and a chemotherapeutic drug in the manufacture of a pharmaceutical composition for treating non-small cell lung cancer, the mixture of antibodies being produced by a single host cell comprising both a nucleic acid encoding the anti-CTLA4 antibody and a nucleic acid encoding the anti-PD1 antibody, wherein the sequence of heavy chain of the anti-CTLA4 antibody is set forth in SEQ ID NO: 17, the sequence of light chain of the anti-CTLA4 antibody is set forth in SEQ ID NO:18, the sequence of heavy chain of the anti-PD1 antibody is set forth in SEQ ID NO:19, and the sequence of light chain of the anti-PD1 antibody is set forth in SEQ ID NO:20; the non-small cell lung cancer is at stage II-IIIB after complete surgical resection; or the non-small cell lung cancer is locally advanced or metastatic non-small cell lung cancer that is negative for PD-L1 expression, has no EGFR-sensitive mutation or altered ALK gene translocation, and has not been subjected to a systemic anti-tumor therapy.
